# EUROPÄISCHE PATENTSCHRIFT

(11) **EP 1 562 893 B1**
(45) Veröffentlichungstag und Bekanntmachung des Hinweises auf die Patenterteilung: **13.01.2010**
(21) Anmeldenummer: 03775352.2
(22) Anmeldetag: 13.11.2003
(51) Int. Cl.: A23G 3/34, A23G 4/06, A23L 1/226, C07C 231/12, C07C 231/02, C07C 233/09

(54) **VERWENDUNG VON TRANS-PELLITORIN ALS AROMASTOFF**
USE OF TRANS-PELLITORIN IN THE FORM OF AN AROMATIC SUBSTANCE
UTILISATION DE TRANS-PELLITORINE COMME SUBSTANCE AROMATIQUE

(30) Priorität: 14.11.2002 DE 10253331
(43) Veröffentlichungstag der Anmeldung: 17.08.2005
(73) Patentinhaber: Symrise GmbH & Co. KG, 37603 Holzminden (DE)
(72) Erfinder: GATFIELD, Ian, Lucas, 37671 Höxter (DE); LEY, Jakob, Peter, 37603 Holzminden (DE); KRAMMER, Gerhard, 37603 Holzminden (DE); BERTRAM, Heinz-Jürgen, 37603 Holzminden (DE); LÖNNEKER, Ilse, 37639 Bevern (DE); MACHINEK, Arnold, 37603 Holzminden (DE)
(74) Vertreter: Eisenführ, Speiser & Partner
(86) Internationale Anmeldenummer: PCT/EP2003/012686
(87) Internationale Veröffentlichungsnummer: WO 2004/043906

(56) Entgegenhaltungen:
- EP-A- 1 323 356
- M.JACOBSON: "Pellitorine Isomers. II The Synthesis of N-Isobutyl-trans-2,trans-4-decadienamide" JOURNAL OF THE AMERICAN CHEMICAL SOCIETY, Bd. 75, 1953, Seiten 2584-2586, XP001179475 in der Anmeldung erwähnt
- R.GAMBOA-LEON, W.S.CHILTON: "<Isobutylamide numbing agents of toothache grass, Ctenium aromaticum" BIOCHEMICAL SYTEMATICS AND ECOLOGY, Bd. 28, 2000, Seiten 1019-1021, XP002270990
- DATABASE CAPLUS [Online] CHEMICAL ABSTRACTS SERVICE, COLUMBUS, OHIO, US; GULLAND, J.M.; HOPTON, G.U.: "Pellitorine, the pungent principle of Anacyclus pyrethrum" XP002270991 Database accession no. 1930:23787

## Beschreibung

Die Erfindung betrifft die Verwendung von 2E,4E-Decadiensäure-N-isobutylamid (trans-Pellitorin) als Aromastoff, insbesondere als speichelfördemder Aromastoff, bevorzugt in der Ernährung, der Mundhygiene oder dem Genuss dienenden Zubereitungen. Ferner betrifft die Erfindung Zubereitungen, Halbfertigwaren sowie Riech, Aroma- und Geschmackstoffkompositionen, enthaltend trans-Pellitorin sowie ein Verfahren zur Herstellung von trans-Pellitorin.

Speichelfördernde Stoffe werden z.B: eingesetzt, um krankhafte Mundtrockenheit zu bekämpfen, den Appetit anzuregen oder auch um die Mundhygiene zu verbessern, in dem durch den verstärkten Speichelfluss schädliche Stoffe oder Keime aus der Mundhöhle ausgespült werden. In Nahrungsmittelzubereitungen werden dazu meist Genusssäuren eingesetzt, z.B. Zitronen-, Wein- oder Äpfelsäure. Spezielle, die Speicheldrüsen anregende Stoffe wie z.B. das aus dem Jaborandi-Baum gewonnene Pilocarpin werden bei krankhafter Mundtrockenheit eingesetzt (Acta Med. Croatica 2000, 54, 65-67). Solche hochpotenten cholinergen Parasympathomimetika haben jedoch meist gravierende Nebenwirkungen und sind teilweise sehr giftig.

Unter den langkettigen Fettsäurealkylamiden wurde 2E,6Z,8E-Decatriensäure-N-isobutylamid (Spilanthol) als stark speichelfördernd und kribbelnd beschrieben. Spilanthol ist aber zugleich vor allem scharf und stark betäubend sowie adstringierend (Lebensm.-Wiss. u. -Technol. 1992, 25, 417-421). Andere, längerkettige Polyensäure-N-isobutylamide oder Polyinsäure-N-isobutylamide scheinen zwar stärker speichelanregend zu sein, zeigen aber einen zusätzlich bitteren Geschmackseindruck (vgl. vorhergehendes Zitat).

Aufgabe der vorliegenden Erfindung war es, einen Stoff mit einem speichelfördernden und/oder kribbelndem Effekt sowie einem an sonsten weitgehend neutralen Aromaprofil zu entwickeln, der als Aromastoff in Zubereitungen eingearbeitet werden kann, insbesondere in der Ernährung, der Mundhygiene oder dem Genuss dienenden Zubereitungen.

Die Erfindung betrifft daher die Verwendung von 2E,4E-Decadiensäureisobutylamid (trans-Pellitorin) als Aromastoff, bevorzugt als Aromastoff mit einem speichelfördernden und/oder kribbelndem Effekt, insbesondere bevorzugt als Aromastoff mit speichelfördernden und/oder kribbelndem Effekt in der Ernährung, der Mundhygiene oder dem Genuss dienenden Zubereitungen gemäß Anspruch 1.

Ein weiterer Gegenstand der vorliegenden Erfindung sind Zubereitungen, Halbfertigwaren und Riech-, Aroma- und Geschmackstofflcompositionen, enthaltend 2E,4E-Decadiensäure-N-isobutylamid, bevorzugt enthaltend synthetisches 2E,4E-Decadiensäure-N-isobutylamid gemäß Anspruch 4.

Das erfindungsgemäße trans-Pellitorin zeigt bei der sensorischen Untersuchung einen angenehmen, speichelanregenden und schwach prickelnden sensorischen Eindruck, der relativ lang anhält. Überraschenderweise ist nur noch ein speichelanregender Effekt zu beobachten, wenn die Konzentration unterhalb von 20 ppm, insbesondere unterhalb von 10 ppm im Endprodukt, beispielsweise einer fertigen Zubereitung, liegt. Dabei sind keine weiteren sensorischen Eindrücke zu erkennen, so dass das Geschmacksprofil sehr neutral ist. Das weitgehend neutrale Geschmacksprofil stellt einen wesentlichen Vorteil bei der Einarbeitung in Zubereitungen dar, da das trans-Pellitorin keine Veränderung oder Verfälschung des Geschmacksprofils der Zubereitung hervorruft.

Dies ist insbesondere deshalb überraschend, da in J. Am. Chem. Soc. 1953, Bd. 75, 2584-2586 bei Verkostung des trans-Pellitorins in purum neben einem erhöhten Speichelfluss eine stark brennende Wirkung auf der Zunge beschrieben ist. Sein sensorischer Eindruck wurde auch als vorwiegend betäubend beschrieben (J. Agric. Food Chem., 1981, Bd. 29, Seiten 115ff. oder Fitoterapia, 2001, Band 72, Seiten 197ff.).

WO2004/000787 beschreibt 10 ppm trans-Pellitorin als speichelfördernd, fettig, fruchtig, leicht kribbelnd und nicht scharf.

Das natürliche Vorkommen von reinem 2E,4E-Decadiensäure-N-isobutylamid wurde vielfach in der Literatur beschrieben; z.B. kommt es in Pfeffer vor (Übersicht G.M. Strunz, Stud. Nat. Prod. Chem. 2000, Band 24 (Bioactive Natural Products (Part E)), 683-738).

Natürliche Produkte, wie z.B. natürliche Extrakte, die trans-Pellitorin enthalten, weisen zusätzliche Aromawirkungen und daher kein neutrales Geschmacksprofil auf. Dies ist eine oftmals unerwünschte Eigenschaft natürlicher Produkte bei der Einarbeitung in Zubereitungen. Daher ist die Verwendung von synthetisiertem, d.h. synthetischem, trans-Pellitorin in der vorliegenden Erfindung bevorzugt.

In einer besonders bevorzugten Ausführung der Erfindung wird 2E,4E-Decadiensäure-N-isobutylamid in Kombination mit anderen speichelfördernden, prickelnd, scharf und/oder warm schmeckenden Substanzen oder pflanzlichen Extrakten verwendet. Auf diese Weise kann gezielt ein besonders abgerundetes sensorisches Profil erreicht werden.

Andere speichelfördernde, prickelnd, scharf und/oder warm schmeckende Substanzen können beispielsweise Genusssäuren (beispielsweise Zitronensäure, Äpfelsäure, Weinsäure), Capsaicin, Dihydrocapsaicin, Gingerol, Paradol, Shogaol, Piperin, Carbonsäure-N-vanillylamide, insbesondere Nonansäure-N-vanillylamid, 2-Alkensäureamide, insbesondere 2-Nonensäure-N-isobutylamid, Spilanthol, 2-Nonensäure-N-4-hydroxy-3-methoxyphenylamid, Allylether von 4-Hydroxy-3-methoxybenzylalkohol, insbesonders 4-Hydroxy-3-methoxybenzyl-n-butylether, Alkylether von 4-Acyloxy-3-methoxybenzylalkohol, insbesondere 4-Acetyloxy-3-methoxybenzyl-n-butylether und 4-Acetyloxy-3-methoxybenzyl-n-hexylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, (4-Hydroxy-3-methoxyphenyl)essigsäureamide, insbesonders (4-Hydroxy-3-methoxyphenyl)essigsäure-N-n-octylamid, Ferulasäurephenethylamiden, Nicotinaldehyd, Methylnicotinat, Propylnicotinat, 2-Butoxyethylnicotinat, Benzylnicotinat, 1-Acetoxychavicol, Polygodial, Isodrimeninol oder Pilocarpin sein.

Das trans-Pellitorin kann vorteilhafterweise mit mindestens einem weiteren N-Isobutylamid aus der Gruppe Decansäure, 2E-Decensäure, 2E,4Z-Decadiensäure, 2Z,4E-Decadiensäure, 2Z,4Z-Decadiensäure, 2E,4Z,7Z-Decatriensäure, 3Z,5E-Decadiensäure oder 3Z,5E,7Z-Decatriensäure verwendet werden. Bevorzugt ist eine Mischung von mindestens 80 Gew.-% 2E,4E-Decadiensäure-N-isobutylamid und höchstens 20 Gew.-% 2E,4Z-Decadiensäure-N-isobutylamid.

Speichelfördernde, prickelnd, scharf und/oder wann schmeckende pflanzliche Extrakte können alle für die Ernährung oder Mundhygiene geeigneten pflanzlichen Extrakte sein, die einen speichelfördernden, prickelnden, scharfen und/oder warmen sensorischen Eindruck hervorrufen. Bevorzugt als pflanzliche Extrakte sind beispielsweise Pfefferextrakt *(Piper ssp.,* insbesondere *Piper nigrum),* Wasserpfefferextrakt *(Polygonum ssp.,* insbesondere *Polygonum hydropiper),* Extrakte aus *Allium ssp.* (insbesondere Zwiebel und Knoblauchextrakte), Extrakte aus Rettich *(Raphanus ssp.),* Meerrettichextrakte *(Cochlearia armoracia),* Extrakte aus schwarzem *(Brassica nigra),* wildem oder gelbem Senf *(Sinapis ssp.,* insbesondere *Sinapis arvensis* und *Sinapis alba),* Bertramwurzel-Extrakte *(Ancyclus ssp.,* insbesondere *Anacylcus pyrethrum* L.), Sonlienhutextrakte *(Echinaceae ssp.),* Extrakte aus Szechuan-Pfeffer *(Zanthoxylum ssp.,* insbesondere *Zanthoxylum piperitum),* Spilanthesextrakt *(Spilanthes ssp.,* insbesondere *Spilanthes acmella),* Chiliextrakt *(Capsicum ssp.,* insbesondere *Capsicum frutescens),* Paradieskörner-Extrakt *(Aframomum ssp.,* insbesondere *Aframomun melegueta* [Rose] K. Schum.), Ingwerextrakt *(Zingiber ssp.,* insbesondere *Zingiber officinale),* Galangaextrakt *(Kaempferia galanga* oder *Alpinia galanga)* und Jaborandi-Extrakt *(Pilocarpus-Spezies,* insbesondere *Pilocarpus jaborandi).*

Die erfindungsgemäßen pflanzlichen Extrakte können aus den entsprechenden frischen oder getrockneten Pflanzen oder Pflanzenteilen, insbesondere aber aus weißen, grünen oder schwarzen Pfefferkörnern, Wasserpfefferkörnern, Zwiebeln und Knoblauch, Rettichwurzel, Meerrettich, Senfkömem, Sonnenhutwurzeln, Bertramwurzel, Pflanzenteilen der *Zanthoxylum-Arten,* Pflanzenteilen der Spilanthes-Arten, Chilischoten, Paradieskörnern oder Ingwer- oder Galangawurzeln sowie Jaborandi gewonnen werden. Üblicherweise werden die getrockneten Pflanzenteile, die vorzugsweise vorher zerkleinert wurden, mit einem für Nahrungs- und Genussmittel geeigneten Lösungsmittel, vorzugsweise Ethanol, Wasser, Hexan oder Heptan oder Ethanol/Wasser-Gemischen, bei 0°C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise mit Wasserdampf behandelt, meist bei Drücken von 0,01 mbar bis Normaldruck, und/oder in einem für Nahrungs- und Genussmittel geeigneten Lösungsmittel aufgenommen werden.

Ein für Nahrungs- und Genussmittel geeignetes Lösungsmittel kann beispielsweise sein: Wasser, Ethanol, Methanol, Propylenglycol, Glycerin, Aceton, Dichlormethan, Essigsäureethylester, Diethylether, Hexan, Heptan, Triacetin, pflanzliche Öle oder Fette, sowie superkritisches Kohlendioxid oder Gemische der vorgenannten Lösungsmittel.

Die bisher in der Literatur beschriebenen Synthesen zu trans-Pellitorin sind vielstufig mit moderaten bis schlechten Ausbeuten (J. Am. Chem. Soc. 1953, Bd. 75, 2584-2586) oder verwenden toxische Reagenzien wie z.B. das toxische Selendioxid (Bull. Chem. Soc. Jpn., 1984, Bd. 57, Seiten 3013ff.).

Die Erfindung offenbart außerdem ein Verfahren zur Herstellung von 2E,4E-Decadiensäure-N-isobutylamid, dadurch gekennzeichnet, dass
a) ein 2E,4E- oder 2E,4Z-Decadiensäureester oder ein Gemisch dieser Ester mit Isobutylamin in Gegenwart eines Katalysators umgesetzt wird und
b) das in Schritt a) gebildete Produkt, gegebenenfalls nach Reinigungsschritten, zum 2E,4E-Decadiensäure-N-isobutylamid isomerisiert wird;
   oder
   i) ein 2E,4Z-Decadiensäureester oder ein Gemisch aus 2E,4E- und 2E,4Z-Decadiensäureester zum 2E,4E-Decadiensäureester isomerisiert wird und
   ii) das in Schritt i) gebildete Produkt, gegebenenfalls nach Reinigungsschritten, mit Isobutylamin in Gegenwart eines Katalysators umgesetzt wird.

Selbstverständlich kann man Schritt i) weglassen und direkt einen 2E,4E-Decadiensäureester in die Umsetzung mit Isobutylamin einsetzen.

Vorteilhaft ist ein Verfahren, dadurch gekennzeichnet, dass die Umsetzung mit Isobutylamin in Gegenwart eines Katalysators, bevorzugt eines Enzyms, insbesondere eines Enzyms mit Lipaseaktivität, wobei das Enzym als freies Protein oder an einen Träger assoziiert vorliegen kann, erfolgt,
man das resultierende Reaktionsgemisch mit nicht umgesetztem 2,4-Decadiensäureester gegebenenfalls einer Verseifung, bevorzugt mit einem Enzym in einem wässrigen Medium oder einer mit Wasser verdünnten Base, insbesondere bevorzugt einer wässrigen Lösung von anorganischen basischen Salzen, unterzieht,
die gebildete 2,4-Decadiensäure, bevorzugt extraktiv, abtrennt,
das gereinigte oder ungereinigte Rohprodukt zum 2E,4E-Decadiensäure-N-isobutylamid isomerisiert und
anschließend das Gemisch mit physikalisch chemischen Methoden, bevorzugt durch Kristallisation, Chromatographie, Destillation oder Codestillation, aufreinigt.

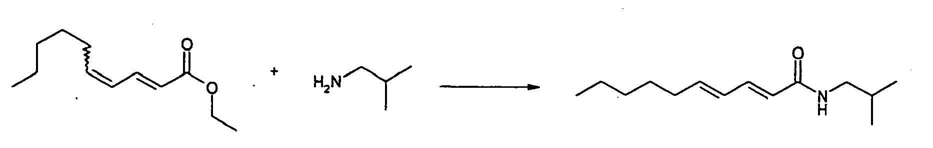

Überraschenderweise wurde festgestellt, dass das offenbarte Verfahren einen sehr einfachen Zugang zu großen, leicht zu reinigenden Mengen des gewünschten 2E,4E-Decadiensäure-N-isobutylamids ermöglicht. Zudem kann so bei Einsatz von natürlichen Reagenzien im Sinne der Aromastoffverordnung natürliches trans-Pellitorin gewonnen werden.

Bei den 2E,4E- oder 2E,4Z-Decadiensäureestern handelt es sich vorteilhafterweise um Ester der 2E,4E- oder 2E,4Z-Decadiensäure mit aliphatischen einwertigen Alkoholen mit 1 bis 20 C-Atomen, insbesondere aber Methanol, Ethanol, 1-Propanol, 2-Propanol, 1-Butanol, 2-Butanol, 2-Methyl-1-propanol, 2-Methyl-2-propanol, 1-Pentanol, 2-Pentanol, 3-Pentanol, 1-Hexanol, 2-Hexanol, 3-Hexanol, 1-Heptanol, 2-Heptanol, 3-Heptanol, 4-Heptanol, 1-Octanol, 2-Octanol, 1-Nonanol, 1-Decanol, 1-Undecanol, 1-Dodecanol, 1-Tridecanol, 1-Tetradecanol, 1-Pentadecanol, 1-Hexadecanol, 1-Heptadecanol, 1-Octadecanol, 1-Nonadecanol, 1-Eicosanol
oder mehrwertigen Alkoholen mit 2 bis 18 C-Atomen wie Ethylenglycol, 1,2-Propylenglycol, 1,3-Propylenglycol, Glycerin, Pentaerythrit, Zuckeralkoholen wie Erythritol, Sorbitol, Glucitol, Mannit, Monosaccharide wie Tetraosen, z.B. Erythrose oder Threose, Pentaosen, z.B. Arabinose, Ribose, Lyxose, Xylose, Hexaosen wie Allose, Altrose, Galactose, Mannose, Gulose, Idose, Glucose, Talose, Fructose, Oligosaccharide wie Maltose, Raffinose, Sucrose, Maltooligosaccharide oder Lactose, wobei die weiteren OH-Gruppen der mehrwertigen Alkohole mit aliphatischen, gesättigten oder ungesättigten Carbonsäuren verestert sein können, sowie deren Gemische oder gesättigter oder ungesättigter Hydroxycarbonsäuren mit 4 bis 20 C-Atomen, die ihrerseits wieder mit den erwähnten Alkoholen verestert sein können.

Vorteilhafte aliphatische, gesättigte oder ungesättigte Carbonsäuren im Sinne der Offenbarung sind gesättigte oder ein- oder mehrfach ungesättigte lineare Carbonsäuren mit 2 bis 26 Kohlenstoffatomen, insbesondere aber Essigsäure, Propionsäure, Buttersäure, Pentansäure, Hexansäure, Heptansäure, Octansäure, Nonansäure, 2E-Nonensäure, Decansäure, 2E-Decensäure, die verschiedenen Isomere der Decadien- oder Decatriensäure, z.B. 2E,4E-Decadiensäure, 2E,4Z-Decadiensäure, 2E,4Z,7Z-Decatriensäure, 3Z,5E-Decadiensäure, 3Z,5E,7Z-Decatriensäure, Deca-2,8-dien-4,6-diinsäure, Deca-2-en-4,6,8-triinsäure, Undecansäure, Dodecansäure, Tridecansäure, Tetradecansäure; Pentadecansäure, Hexadecansäure, 9E- oder 9Z-Hexadecensäure, Heptadecansäure, Octadecansäure, 9E- oder 9Z- oder 11 E- oder 11Z-Octadecensäure, die verschiedenen geometrischen Isomeren der 9,12-Octadecadiensäure, der 6,9,12-Octadecatriensäure, der 9,12,15-Octadecatriensäure, der 6,9,12,15-Octadecatetraensäure, die Nonadecansäure, die Eicosansäure, die verschiedenen geometrischen Isomeren der Eicosaensäure, der 11,14-Eicosadiensäure, der 8,11,14-Eicosatriensäure, der 5,8,11,14-Eicosatetraensäure, der 5,8,11,14,17-Eicosapentaensäure, der 10,13,16-Docosatriensäure, der 7,10,13,16-Docosatetraensäure, der 4,7,10,13,16-Docosapentaensäure und der 4,7,10,13,16,19-Docosahexaensäure.

Die 2,4-Decadiensäureester im Sinne der Offenbarung können bevorzugt in Form natürlicher oder angereicherter prozessierter Triglyceride, beispielsweise aus Stillingiaöl, oder als Methyl- oder Ethylester vorliegen. Besonders bevorzugt ist ein durch enzymatische Umesterung von Stillingiaöl in Ethanol und anschließende Destillation erhaltene Fraktion, dadurch gekennzeichnet, dass sie mindestens 80 Gew.-% Ethyl-2E,4Z-decadienoat enthält.

Isomerisierung im Sinne der Offenbarung bedeutet, dass die anderen, nicht dem trans-Pellitorin entsprechenden möglichen geometrische Isomere des 2,4-Decadiensäure-Restes (2E,4Z-, 2Z,4Z- oder 2Z,4E-) durch an sich bekannte Methoden zum 2E,4E-Isomer umgewandelt werden. Bevorzugt wird das 2E,4Z-Isomer durch Behandlung mit Iod oder durch Bestrahlung mit UV-Licht (Wellenlänge zwischen 250 und 320 nm) in das 2E,4E-Isomer umgewandelt. Die Isomerisierung kann an den 2,4-Decadiensäure-N-isobutylamiden oder den 2,4-Decadiensäureestem durchgeführt werden.

Weiterer Gegenstand der Erfindung sind der Ernährung oder dem Genuss dienende Zubereitungen, enthaltend 2E,4E-Decadiensäure-N-isobutylamid in einer wirksamen Menge gemäß Anspruch 4 und gegebenenfalls andere übliche Grund-, Hilfs- und Zusatzstoffe für Nahrungs- und Genussmittel. Diese Zubereitungen enthalten besonders bevorzugt 0,00001 Gew.-% (0,1 ppm) bis 0,0015 Gew.-% (15 ppm) an 2E,4E-Decadiensäure-N-isobutylamid, bezogen auf das Gesamtgewicht der Zubereitung. Weitere übliche Grund-, Hilfs- und Zusatzstoffe für Nahrungs- oder Genussmittel können in Mengen von 0,000001 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die der Ernährung oder dem Genuss dienenden Zubereitungen im Sinne der Erfindung sind z.B. Backwaren (z.B. Brot, Trockenkekse, Kuchen, sonstiges Gebäck; Süßwaren (z.B. Schokoladen, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke, Fleischprodukte (z.B. Schinken, Frischwurst- oder Rohwurstzubereitungen), Eier oder Eiprodukte (Trockenei, Eiweiß, Eigelb), Getreideprodukte (z.B. Frühstückscerealien, Müsliriegel), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen), Gemüsezubereitungen (z.B. Ketchup, Soßen, Trockengemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), Fertiggerichte und Suppen, Gewürze, Würzmischungen sowie insbesondere Aufstreuwürzungen (Seasonings), die im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Ein besonders bevorzugte Ausführung der Erfindung sind der Mundhygiene dienende Zubereitungen, insbesondere Zahnpflegemittel wie Zahnpasten, Zahngele, Zahnpulver, Mundwässer, Kaugummis und andere Mundpflegemittel, enthaltend 2E,4E-Decadiensäure-N-isobutylamid in einer wirksamen Menge und gegebenenfalls andere übliche Grund-, Hilfs- und Zusatzstoffe für solche Zubereitungen. Sie enthalten besonders bevorzugt 0,00001 Gew.-% (0,1 ppm) bis 0,0015 Gew.-% (15 ppm), bezogen auf das Gesamtgewicht der Zubereitung, an 2E,4E-Decadiensäure-N-isobutylamid. Weitere übliche Grund-, Hilfs- und Zusatzstoffe für die der Mundhygiene dienenden Zubereitungen können in Mengen von 0,000001 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Zahnpflegemittel, enthaltend 2E,4E-Decadiensäure-N-isobutylamid, bestehen im allgemeinen aus einem abrasiven System (Schleif- oder Poliermittel), wie z.B. Kieselsäuren, Calciumcarbonaten, Calciumphosphaten, Aluminiumoxiden und/oder Hydroxylapatiten, aus oberflächenaktiven Substanzen, wie z.B. Natriumlaurylsulfat, Natriumlaurylsarcosinat und/oder Cocamidopropylbetain, aus Feuchthaltemitteln, wie z.B. Glycerin und/oder Sorbit, aus Verdickungsmitteln, wie z.B. Carboxymethylcellulose, Polyethylenglycolen, Carrageenanen und/oder Laponiten^{®}, aus Süßstoffen, wie z.B. Saccharin, aus Stabilisatoren und aus aktiven Wirkstoffen, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat.

Kaugummis, enthaltend 2E,4E-Decadiensäure-N-isobutylamid, bestehen im allgemeinen aus einer Kaugummibase, d.h. einer beim Kauen plastische werdenden Kaumasse, aus Zuckern verschiedener Arten, Zuckeraustauschstoffen, Süßstoffen, Zuckerallcoholen, Feuchthaltemitteln, Verdickern, Emulgatoren, Aromen und Stabilisatoren.

Bevorzugt können erfindungsgemäße Zubereitungen auch in Aufstreuwürzungen, sogenannten Seasonings, eingesetzt werden, um das trockene Mundgefühl, das beim Verzehr von Mais-, Kartoffel- oder Reismehl-Chips und -Snacks entsteht, zu vermeiden und den sensorischen Gesamteindruck zu verbessern.

Geeignete Aufstreuwürzungen enthalten z.B. synthetische, natürliche oder naturidentische Aromastoffe sowie Trägerstoffe wie z.B. Maltodextrin, Salze wie z.B. Kochsalz, Gewürze wie z.B. Paprika und Pfeffer, Zuckerstoffe wie z.B. Saccharin und Geschmacksverstärker wie z.B. Mononatriumglutamat und/oder Inosinmonophosphat.

Die erfindungsgemäßen Zubereitungen, enthaltend 2E,4E-Decadiensäure-N-isobutylamid, können dergestalt hergestellt werden, dass 2E,4E-Decadiensäure-N-isobutylamid als Substanz, als Lösung oder in Form eines Gemisches mit einem festen oder flüssigen Trägerstoff in die der Ernährung, der Mundhygiene oder dem Genuss dienenden Zubereitungen eingearbeitet werden. Vorteilhafterweise können die als Lösungen vorliegenden erfindungsgemäßen Zubereitungen, enthaltend 2E,4E-Decadiensäure-N-isobutylamid, auch durch Sprühtrocknung in eine feste Zubereitung überführt werden.

Zur Herstellung der Zubereitungen können in einer weiteren bevorzugten Ausführungsform die 2E,4E-Decadiensäure-N-isobutylamid und gegebenenfalls andere Bestandteile der erfindungsgemäßen Zubereitung auch vorher in Emulsionen, in Liposomen, z.B. ausgehend von Phosphatidylcholin, in Microsphären, in Nanosphären oder auch in Kapseln aus einer für Lebens- und Genussmittel geeigneten Matrix, z.B. aus Stärke, Stärkederivaten, anderen Polysacchariden, natürlichen Fetten, natürlichen Wachsen oder aus Proteinen, z.B. Gelatine, eingearbeitet werden. Eine weitere Ausführungsform besteht darin, dass 2E,4E-Decadiensäure-N-isobutylamid vorher mit geeigneten Komplexbildnern, beispielsweise mit Cyclodextrinen oder Cyclodextrinderivaten, bevorzugt -Cyclodextrin, komplexiert werden und in dieser Form eingesetzt werden.

Als andere Bestandteile für die erfindungsgemäßen, der Ernährung oder dem Genuss dienenden Zubereitungen können weitere übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Ei, Knochen, Knorpel, Fisch, Krusten- und Schalentiere, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit, Mannitol, Xylitol), natürliche oder gehärtete Fette (z.B. Talg, Schmalz, Palmfett, Kokosfett, gehärtetes Pflanzenfett), fette Öle (z.B. Sonnenblumenöl, Erdnussöl, Maiskeimöl, Distelöl, Olivenöl, Walnussöl, Fischöl, Sojaöl, Sesamöl), Fettsäuren oder deren Salze (z.B. Kaliumstearat, Kaliumpalmitat), proteinogene oder nicht-proteinogene Aminosäuren und verwandte Verbindungen (z.B. Taurin, Kreatin, Kreatinin), Peptide, native oder prozessierte Proteine (z.B. Gelatine), Enzyme (z.B. Peptidasen, Glucosidasen, Lipasen), Nukleinsäuren, Nucleotide (Inositolphosphat), geschmacksmodulierende Stoffe (z.B. Natriumglutamat, 2-Phenoxypropionsäure), Emulgatoren (z.B. Lecithine, Diacylglycerole), Stabilisatoren (z.B. Carageenan, Alginat, Johannisbrotkernmehl, Guarkemmehl), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin), Süßstoffe (z.B. Saccharin, Cyclamat, Aspartam, Neotam, Neohesperidindihydrochalkon), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, sowie Riechstoffe, synthetische, natürliche oder naturidentische Aroma- und Geschmackstoffe.

Bevorzugt können die erfindungsgemäßen Zubereitungen auch noch eine Aromakomposition enthalten, um den Geschmack und/oder Geruch der Zubereitung abzurunden und zu verfeinern. Geeignete Aromakompositionen enthalten z.B. synthetische, natürliche oder naturidentische Aromastoffe sowie Riechstoffe, insbesondere aber auch andere speichelfördernde, prickelnd, scharf oder warm schmeckende Substanzen oder Pflanzenextrakte.

Ein weiterer Gegenstand der Erfindung ist die Verwendung der erfindungsgemäßen Zubereitungen als Halbfertigwaren zur Aromatisierung von daraus gefertigten Zubereitungen als Fertigwaren gemäß Anspruch 10.

Offenbart werden zudem Riech-, Aroma- und Geschmackstoffkompositionen, enthaltend 2E,4E-Decadiensäure-N-isobutylamid. Diese Kompositionen enthalten in der Regel 0,001 Gew.-% bis 5 Gew.-%, bevorzugt 0,01 Gew.-% bis 2 Gew.-%, besonders bevorzugt bevorzugt 0,05 Gew.-% bis 1 Gew.-% an 2E,4E-Decadiensäure-N-isobutylamid, bezogen auf das Gesamtgewicht der Komposition.

Die Erfindung kann anhand der folgenden Beispiele erläutert werden.

### Beispiele

### Beispiel 1:

### 2E,4E-Decadiensäure-N-isobutylamid (trans-Pellitorin) durch enzymatische Umsetzung mit Ethyl-2E,4Z-decadienoat mit anschließender Isomerisierung

### Umsetzung mit Isobutylamin:

10 g Ethyl-2E,4Z-decadienoat, 4,7 g Chirazym L-2 (c.-f., C2, lyo., Katalog-Nr. 1859242, Roche Diagnostics, Basel, Schweiz) und 4 g Isobutylamin wurden bei 55°C 4 Tage gerührt. Der Ansatz wurde mit 100 ml Diethylether versetzt und filtriert; das Filtrat wurde im Vakuum eingedampft (Rohausbeute 15,2 g). Das Produkt wurde in 10 %iger KOH/Methanol (1:1-Gemisch) 45 min bei Raumtemperatur gerührt, mit Ether extrahiert, die etherische Phase über Natriumsulfat getrocknet, filtriert und das Filtrat eingedampft. Das rohe Zwischenprodukt wurde an Kieselgel 60 chromatographiert (Eluent Hexan/Ethylacetat 10:1 (v/v)). Ausbeute 9,1 g (GC: 99,4 %).
¹H-NMR (CDCl₃; .200 MHz): δ = 7,56 (1H, ddd, 11,5 Hz, 14,9 Hz, 1.0 Hz), 6,08 (1H, dddd 11,5 Hz, 10,8 Hz, 1,4 Hz, 0,6 Hz), 5,82 (1H, d, 14,9 Hz), 5,79 (1H, dtd 10,8 Hz, 7,8 Hz, 0,9 Hz), 5,50 (1H, bs), 3,18 (2H, dd, 6,8 Hz, 6,1 Hz), 2,36-2,22 (2H, m), 1,81 (1H, m, 6,7 Hz), 1,50 - 1,22 (6H,m), 0,93 (6H, d, 6,7 Hz); 0,88 (3H, m) ppm.
¹³C-NMR (CDCl₃; 50 MHz): δ = 166,34 (C), 140,07 (CH), 135,76 (CH), 126,28 (CH), 123,78 (CH), 46,96 (CH₂), 31,41 (CH₂), 29,14 (CH₂), 28,63 (CH), 28,15 (CH₂), 22,52 (CH₂), 20,15 (CH₃), 14,02 (CH₃) ppm.

### Isomerisierung:

277 mg des gereinigten 2E,4Z-Decadiensäure-N-isobutylamids wurden mit 29 mg Iod in 10 ml Toluol eine Stunde bei Raumtemperatur gerührt. Die Mischung wurde an Kieselgel 60 mit dem Eluenten Hexan/Ethylacetat 5:1 (v/v) chromatographiert. Ausbeute: 61 mg (Reinheit > 95 %, NMR).
¹H-NMR (CDCl₃; 200 MHz): δ = 7,19 (1H, dd, 14,9 Hz, 9,7 Hz), 6,13 (1H, dd 15,1 Hz, 9,6 Hz), 6,07 (1H, dd, 15,1 Hz, 6,4 Hz), 5,75 (1H, d 14,9 Hz), 5,50 (1H, bs), 3,17 (2H, dd, 6,9 Hz, 6,1 Hz), 2,14 (2H, dd, 7Hz, 6,4 Hz), 1,80 (1H, m, 6,7 Hz), 1,42 (2H, m, 7,1 Hz), 1,37 - 1,22 (4H, m), 0,93 (6H, d, 6,7 Hz), 0,89 (3H, m) ppm.

### Isomerisierung Variante 1:

3,1 g des rohen Zwischenprodukts aus der Umsetzung mit Isobutylamin werden in 20 ml Toluol mit 60 mg Iod versetzt und 26 h bei Raumtemperatur gerührt. Es werden 30 ml n-Hexan zugegeben und das Gemisch bei ca. 18°C 1 h gelagert. Das kristalline Produkt wird abfiltriert (GC: 86 % 2E,4E-Isomer, 10,5 % 2E,4Z-Isomer). Durch Umkristallisation aus ca. 30 ml n-Hexan kann ein 95 %iges Produkt gewonnen werden (Ausbeute 1,6 g).

### Isomerisierung Variante 2:

3,1 g des rohen Zwischenprodukts aus der Umsetzung mit Isobutylamin werden in 100 ml Ethanol gelöst und unter Kühlung mit einem Quecksilberhochdruckstrahler mit Quarzglas-Tauchrohr 8 Stunden bestrahlt. Die Lösung wird eingedampft und der ölige Rückstand (GC: 22 % 2E,4E-Isomer, 62 % 2E,4Z-Isomer) chromatographisch aufgereinigt (Ausbeute ca. 400 mg).

### Beispiel 2: Verkostung

Das trans-Pellitorin wurde in Ethanol gelöst und die ethanolische Lösung dann mit 11 Gew.-%iger Zuckerlösung verdünnt (Endkonzentration: c). Zur Verkostung wurden jeweils ca. 5 ml der Zuckerlösung heruntergeschluckt. Eine Gruppe von 6 bis 8 Prüfern hat die Lösung verkostet:
- c = 10 ppm:: speichelfördernd, leicht kribbelnd, nicht scharf
- c = 20 ppm:: speichelfördernd, leicht betäubend, leicht fettig, schwach fruchtig, kribbelnd, lang anhaltend, nicht scharf

### Beispiel 3: Anwendung in einer Zahnpasta

| **Teil** | **Inhaltsstoff** | **Einsatz in Gew.=%** |
|---|---|---|
| A | demineralisiertes Wasser | 22,00 |
| | Sorbitol (70 %) | 45,00 |
| | Solbrol^{®} M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Aromakomposition, enthaltend 0,1 Gew.-% 2E,4E-Decadiensäure-N-isobutylamid | 1 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C 30 min gut verrührt. Nach Entspannung ist die Zahnpasta fertig und kann abgefüllt werden.

### Beispiel 4: Anwendung in einem zuckerfreien Kaugummi

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70 % | 14,00 |
| | Glycerin | 1,00 |
| D | Aromakomposition, enthaltend 0,1 Gew.-% 2E,4E-Decadiensäure-N-isobutylamid | 1 |

Teile A bis D werden gemischt und intensiv geknetet. Die Rohmasse kann z.B. in Form von dünnen Streifen zu verzehrfertigen Kaugummis verarbeitet werden.

### Beispiel 5: Anwendung in einem Mundwasser

| **Teil** | **Inhaltsstoff** | **Gehalt (Gew.-%)** |
|---|---|---|
| A | Ethanol | 10,00 |
| | Cremophor^{®} CO 40 (BASF, Detergenz) | 1,00 |
| | Benzoesäure | 0,12 |
| | Aroma, enthaltend 0,4 Gew.-% 2E,4E-Decadiensäure-N-isobutylamid | 0,25 |
| B | demineralisiertes Wasser | 83,46 |
| | Sorbitol, 70 % | 5,00 |
| | Natriumsaccharin 450 | 0,07 |
| | L-Blue 5000 e.c., 1 Gew.-% in Wasser (Farbstoff) | 0,10 |

Die Inhaltsstoffe der Teile A und B werden jeweils für sich gemischt. Teil B wird langsam in Teil A eingerührt, bis die Mischung homogen ist.

### Beispiel 6: Anwendung in einer Aufstreuwürze für frittiertes Knabbergebäck

100 g nicht gewürzte Tortilla Chips werden mit einer Mischung aus 7 g Käse Trockenaroma für Snacks und 0,07 g 2E,4E-Decadiensäure-N-isobutylamid überstreut.

### Beispiel 7: Anwendung in einer Kekscremefüllung

100 g Standard-Cremefüllung werden mit 0,4 g Erdbeer-Aroma und 0,1 g 2E,4E-Decadiensäure-N-isobutylamid intensiv vermischt.

## Patentansprüche

1. Verwendung von 2E,4E-Decadiensäureisobutylamid (trans-Pellitorin) in einer (a) der Mundhygiene dienenden Zubereitung oder (b) der Ernährung oder dem Genuss dienenden Zubereitung,
**dadurch gekennzeichnet, dass** trans-Pellitorin in einer Konzentration 0,01 bis 20 ppm bezogen auf die fertige Zubereitung vorliegt,
mit der Maßgabe, dass die Zubereitung keine ethanolische Lösung darstellt, die mit 11 %iger Zuckerlösung verdünnt ist und 10 ppm trans-Pellitorin bezogen auf die fertige Zubereitung enthält.

2. Verwendung nach Anspruch 1, **dadurch gekennzeichnet, dass** die der Ernährung oder dem Genuss dienende Zubereitung ausgewählt wird aus der Gruppe bestehend aus Backwaren, bevorzugt Brot, Trockenkekse, Kuchen, sonstiges Gebäck; Süßwaren, bevorzugt Schokoladen, Fruchtgummi, Hart- und Weichkaramellen, Kaugummi; alkoholische oder nichtalkoholische Getränke, bevorzugt Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen, Instantgetränke; Fleischprodukte, bevorzugt Schinken, Frischwurst- oder Rohwurstzubereitungen; Eier oder Eierprodukte, bevorzugt Trockenei, Eiweiß, Eigelb; Getreideprodukte, bevorzugt Frühstückscerealien, Müsliriegel; Milchprodukte, bevorzugt Milchgetränke, Milcheis, Joghurt, Kefir, Frischkäse, Weichkäse, Hartkäse, Trockenmilchpulver, Molke, Butter, Buttermilch; Fruchtzubereitungen, bevorzugt Konfitüren, Fruchteis, Fruchtsoßen; Gemüsezubereitungen, bevorzugt Ketchup, Soßen, Trockengemüse; Knabberartikel, bevorzugt gebackene oder frittierte Kartoffelchips oder Kartoffelteigprodukte, Extrudate auf Mais- oder Erdnussbasis; Produkte auf Fett- und Ölbasis oder Emulsionen derselben, bevorzugt Mayonnaise, Remoulade, Dressings; Fertiggerichte und Suppen; Gewürze, Würzmischungen sowie insbesondere Aufstreuwürzungen (Seasonings); Kapseln, nichtüberzogene sowie überzogene Tabletten, bevorzugt mit magensaftresistenten Überzügen; Dragees; Granulaten, Pellets, Feststoffmischungen; Dispersionen in flüssigen Phasen, Emulsionen, Pulver; Lösungen; Pasten und andere schluck- oder kaubare Zubereitungen als Nahrungsergänzungsmittel.

3. Verwendung gemäß Anspruch 1 oder 2,
**dadurch gekennzeichnet, dass** trans-Pellitorin in einer Konzentration von 0,1 ppm bis 15 ppm bezogen auf die fertige Zubereitung vorliegt.

4. Zubereitung, die (a) der Mundhygiene oder (b) der Ernährung oder dem Genuss dient,
**dadurch gekennzeichnet, dass** die Zubereitung trans-Pellitorin in einer Konzentration 0,01 bis 20 ppm bezogen auf die fertige Zubereitung umfasst,
mit der Maßgabe, dass die Zubereitung keine ethanolische Lösung darstellt, die mit 11 %iger Zuckerlösung verdünnt ist und 10 ppm trans-Pellitorin bezogen auf die fertige Zubereitung enthält.

5. Zubereitung, die der Ernährung oder dem Genuss dient nach Anspruch 4,
**dadurch gekennzeichnet, dass** die Zubereitung ausgewählt wird aus der Gruppe bestehend aus Backwaren, bevorzugt Brot, Trockenkekse, Kuchen, sonstiges Gebäck; Süßwaren, bevorzugt Schokoladen, Fruchtgummi, Hart- und Weichkaramellen, Kaugummi; alkoholische oder nichtalkoholische Getränke, bevorzugt Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen, Instantgetränke; Fleischprodukte, bevorzugt Schinken, Frischwurst- oder Rohwurstzubereitungen; Eier oder Eierprodukte, bevorzugt Trockenei, Eiweiß, Eigelb; Getreideprodukte, bevorzugt Frühstückscerealien, Müsliriegel; Milchprodukte, bevorzugt Milchgetränke, Milcheis, Joghurt, Kefir, Frischkäse, Weichkäse, Hartkäse, Trockenmilchpulver, Molke, Butter, Buttermilch; Fruchtzubereitungen, bevorzugt Konfitüren, Fruchteis, Fruchtsoßen; Gemüsezubereitungen, bevorzugt Ketchup, Soßen, Trockengemüse; Knabberartikel, bevorzugt gebackene oder frittierte Kartoffelchips oder Kartoffelteigprodukte, Extrudate auf Mais- oder Erdnussbasis; Produkte auf Fett- und Ölbasis oder Emulsionen derselben, bevorzugt Mayonnaise, Remoulade, Dressings; Fertiggerichte und Suppen; Gewürze, Würzmischungen sowie insbesondere Aufstreuwürzungen (Seasonings); Kapseln, nichtüberzogene sowie überzogene Tabletten, bevorzugt mit magensaftresistenten Überzügen; Dragees; Granulaten, Pellets, Feststoffmischungen; Dispersionen in flüssigen Phasen, Emulsionen, Pulver; Lösungen; Pasten und andere schluck- oder kaubare Zubereitungen als Nahrungsergänzungsmittel.

6. Zubereitung nach Anspruch 4 oder 5,
**dadurch gekennzeichnet, dass** die Konzentration von trans-Pellitorin bezogen auf die fertige Zubereitung zwischen 0,1 ppm und 15 ppm liegt.

7. Zubereitung nach einem der Ansprüche 4 bis 6,
**dadurch gekennzeichnet, dass** diese mindestens eine weitere speichelfördernde, prickelnde, scharf- oder warmschmeckende Substanz enthält.

8. Zubereitungen nach mindestens einem der Ansprüche 4 bis 7,
**dadurch gekennzeichnet, dass** diese mindestens einen speichelfördernden, prickelnden, scharf- oder warmschmeckenden pflanzlichen Extrakt enthalten.

9. Zubereitungen nach mindestens einem der Ansprüche 4 bis 8,
**dadurch gekennzeichnet, dass** es sich um synthetisches trans-Pellitorin handelt.

10. Verwendung der Zubereitung, die der Ernährung oder dem Genuss dient, nach einem der Ansprüche 4 bis 9, als Halbfertigware zur Aromatisierung von daraus gefertigten Zubereitungen als Fertigwaren, wobei die Fertigwaren eine Konzentration von trans-Pellitorin im Bereich von 0,01 bis 20 ppm haben.

## Claims

1. Use of 2E,4E-decadienoic acid isobutylamide (trans-pellitorin) in a (a) formulation for oral hygiene or (b) formulation for nutrition or consumption for pleasure,
**characterized in that** trans-pellitorin is present in a concentration of 0.01 to 20 ppm, based on the finished formulation,
with the proviso that the formulation is not an ethanolic solution which is diluted with 11 % strength sugar solution and contains 10 ppm of trans-pellitorin, based on the finished formulation.

2. Use according to claim 1, **characterized in that** the formulation for nutrition or consumption for pleasure is chosen from the group consisting of baked goods, preferably bread, dry biscuits, cakes, other baked products; confectionery, preferably chocolate, fruit gum, hard and soft caramels, chewing gum; alcoholic or non-alcoholic drinks, preferably coffee, tea, wine, wine-containing drinks, beer, beer-containing drinks, liqueurs, schnapps, brandies, fruit-containing carbonated drinks, isotonic drinks, soft drinks, nectars, fruit and vegetable juices, fruit or vegetable juice formulations, instant drinks; meat products, preferably ham, fresh sausage or uncooked sausage formulations; eggs or egg products, preferably dried egg, egg white, egg yolk; cereal products, preferably breakfast cereals, muesli bars; dairy products, preferably milk drinks, milk ice, yoghurt, kefir, fresh cheese, soft cheese, hard cheese, dried milk powder, whey, butter, buttermilk; fruit formulations, preferably preserves, fruit-flavoured ice-cream, fruit sauces; vegetable formulations, preferably ketchup, sauces, dried vegetables; nibbles, preferably baked or fried potato chips or potato paste products, extrudates based on maize or peanuts; fat- or oil-based products or emulsions thereof, preferably mayonnaise, remoulade, dressings; ready-made dishes and soups; spices, spice mixtures and, in particular, sprinkling spices (seasonings); capsules, non-coated and coated tablets, preferably with coatings resistant to gastric juice; lacquered tablets; granules, pellets, mixtures of solids; dispersions in liquid phases, emulsions, powders; solutions; pastes and other formulations, as food supplements, which can be swallowed or chewed.

3. Use according to claim 1 or 2,
**characterized in that** trans-pellitorin is present in a concentration of from 0.1 ppm to 15 ppm, based on the finished formulation.

4. Formulation for (a) oral hygiene or (b) nutrition or consumption for pleasure,
**characterized in that** the formulation contains trans-pellitorin in a concentration of 0.01 to 20 ppm, based on the finished formulation,
with the proviso that the formulation is not an ethanolic solution which is diluted with 11 % strength sugar solution and contains 10 ppm of trans-pellitorin, based on the finished formulation.

5. Formulation for nutrition or consumption for pleasure according to claim 4,
**characterized in that** the formulation is chosen from the group consisting of baked goods, preferably bread, dry biscuits, cakes, other baked products; confectionery, preferably chocolate, fruit gum, hard and soft caramels, chewing gum; alcoholic or non-alcoholic drinks, preferably coffee, tea, wine, wine-containing drinks, beer, beer-containing drinks, liqueurs, schnapps, brandies, fruit-containing carbonated drinks, isotonic drinks, soft drinks, nectars, fruit and vegetable juices, fruit or vegetable juice formulations, instant drinks; meat products, preferably ham, fresh sausage or uncooked sausage formulations; eggs or egg products, preferably dried egg, egg white, egg yolk; cereal products, preferably breakfast cereals, muesli bars; dairy products, preferably milk drinks, milk ice, yoghurt, kefir, fresh cheese, soft cheese, hard cheese, dried milk powder, whey, butter, buttermilk; fruit formulations, preferably preserves, fruit-flavoured ice-cream, fruit sauces; vegetable formulations, preferably ketchup, sauces, dried vegetables; nibbles, preferably baked or fried potato chips or potato paste products, extrudates based on maize or peanuts; fat- or oil-based products or emulsions thereof, preferably mayonnaise, remoulade, dressings; ready-made dishes and soups; spices, spice mixtures and, in particular, sprinkling spices (seasonings); capsules, non-coated and coated tablets, preferably with coatings resistant to gastric juice; lacquered tablets; granules, pellets, mixtures of solids; dispersions in liquid phases, emulsions, powders; solutions; pastes and other formulations, as food supplements, which can be swallowed or chewed.

6. Formulation according to claim 4 or 5,
**characterized in that** the concentration of trans-pellitorin, based on the finished formulation, is between 0.1 ppm and 15 ppm.

7. Formulation according to one of claims 4 to 6,
**characterized in that** this contains at least one further salivant, tingling, piquant- or warm-tasting substance.

8. Formulations according to at least one of claims 4 to 7,
**characterized in that** these contain at least one salivant, tingling, piquant- or warm-tasting plant extract.

9. Formulations according to at least one of claims 4 to 8,
**characterized in that** the trans-pellitorin is synthetic trans-pellitorin.

10. Use of the formulation for nutrition or consumption for pleasure according to one of claims 4 to 9 as a semi-finished product for aromatizing formulations produced therefrom as finished products, wherein the finished products have a concentration of trans-pellitorin in the range of from 0.01 to 20 ppm.

## Revendications

1. Utilisation de l'isobutylamide de l'acide 2E,4E-décadiénoïque (trans-pellitorine) dans une préparation servant (a) à l'hygiène buccale ou (b) à l'alimentation ou à la stimulation,
**caractérisée en ce que** la trans-pellitorine est présente en une concentration de 0.01 à 20 ppm, rapporté à la composition finie,
avec la condition que la préparation ne représente pas une solution éthanolique diluée par une solution de sucre à 11 % et contenant 10 ppm de trans-pellitorine, rapporté à la composition finie.

2. Utilisation selon la revendication 1, **caractérisée en ce que** la préparation servant à l'alimentation ou à la stimulation est choisie parmi le groupe constitué par les produits de boulangerie, de pâtisserie et de biscuiterie, de préférence le pain, les biscuits secs, les gâteaux, les autres produits de boulangerie, pâtisserie et biscuiterie ; les produits sucrés, de préférence le chocolat, la gomme fruitée, les caramels durs et mous, les chewing-gums ; les boissons alcooliques ou non alcooliques, de préférence le café, le thé, le vin, les boissons à base de vin, la bière, les boissons à base de bière, les liqueurs, les eaux-de-vie, les brandys, les limonades fruitées, les boissons isotoniques, les boissons rafraîchissantes, les nectars, des jus de fruits et de légume, les préparations de jus de fruits ou de légume, les boissons instantanées ; les produits de viande, de préférence le jambon, les charcuteries cuites et crues ; les oeufs ou les produits à base d'oeufs, de préférence les oeufs déshydratés, le blanc d'oeuf, le jaune d'oeuf; les produits de céréales, de préférence les céréales de petit déjeuner, les barres de céréales ; les produits laitiers, de préférence les boissons lactées, le riz au lait, les yaourts, le kéfir, les fromages frais, les fromages à pâte molle, les fromages à pâte cuite, le lait en poudre, petit-lait, le beurre, le babeurre ; les préparations de fruits, de préférence les confitures, les glaces aux fruits, les sauces de fruits ; les préparations de légumes, de préférence les ketchups, les sauces, les légumes déshydratés; les articles d'apéritif, de préférence les pommes chips au four ou frites, ou les produits à base de pâte de pomme de terre, les préparations extrudées à base de maïs ou d'arachide ; les produits à base de graisse et d'huile ou les émulsions de ces dernières, de préférence les mayonnaises, les rémoulades, les dressings ; les plats cuisinés et les soupes ; les condiments, les mélanges de condiments ainsi qu'en particulier les condiments à saupoudrer (seasonings) : les capsules, les comprimés enrobés ou non enrobés, de préférence avec des revêtements résistant au suc gastrique ; les dragées, les granulés, les pellets, les mélanges de corps solides ; les dispersions dans des phases liquides, les émulsions, les poudres, les solutions ; les pâtes et d'autres préparations à avaler ou à mâcher comme compléments alimentaires.

3. Utilisation selon la revendication 1 ou 2,
**caractérisée en ce que** la txans-pellitorine est présente en une concentration de 0,1 ppm à 15 ppm, rapporté à la préparation finie.

4. Préparation servant (a) à l'hygiène buccale ou (b) à l'alimentation ou à la stimulation,
**caractérisée en ce que** la préparation comprend la trans-pellitorine en une concentration de 0,01 à 20 ppm, rapporté à la préparation finie,
avec la condition que la composition ne représente pas une solution éthanolique diluée par une solution de sucre à 11 % et contenant 10 ppm de trans-pellitorine, rapporté à la composition finie.

5. Préparation servant à l'alimentation ou à la stimulation selon la revendication 4,
**caractérisée en ce que** la préparation est choisie parmi le groupe constitué par les produits de boulangerie, de pâtisserie et de biscuiterie, de préférence le pain, les biscuits secs, les gâteaux, les autres produits de boulangerie, pâtisserie et biscuiterie ; les produits sucrés, de préférence le chocolat, la gomme fruitée, les caramels durs et mous, les chewing-gums ; les boissons alcooliques ou non alcooliques, de préférence le café, le thé, le vin, les boissons à base de vin, la bière, les boissons à base de bière, les liqueurs, les eaux-de-vie, les brandys, les limonades fruitées, les boissons isotoniques, les boissons rafraîchissantes, les nectars, des jus de fruits et de légume, les préparations de jus de fruits ou de légume, les boissons instantanées ; les produits de viande, de préférence le jambon, les charcuteries cuites et crues ; les oeufs ou les produits à base d'oeufs, de préférence les oeufs déshydratés, le blanc d'oeuf, le jaune d'oeuf; les produits de céréales, de préférence les céréales de petit déjeuner, les barres de céréales ; les produits laitiers, de préférence les boissons lactées, le riz au lait, les yaourts, le kéfir, les fromages frais, les fromages à pâte molle, les fromages à pâte cuite, le lait en poudre, le petit-lait, le beurre, le babeurre , les préparations de fruits, de préférence les confitures, les glaces aux fruit, les sauces de fruits ; les préparations de légumes, de préférence les ketchups, les sauces, les légumes déshydratés ; les articles d'apéritif, de préférence les pommes chips au four ou frites, ou les produits à base de pâte de pomme de terre, les préparations extrudées à base de maïs ou d'arachide ; les produits à base de graisse et d'huile ou les émulsions de ces dernières, de préférence les mayonnaises, les rémoulades, les dressings ; les plats cuisinés et les soupes ; les condiments, les mélanges de condiments ainsi qu'en particulier les condiments à saupoudrer (seasonings) ; les capsules, les comprimés enrobés ou non enrobées, de préférence avec des revêtements résistant au suc gastrique ; les dragées, les granulés, les pellets, les mélanges de corps solides ; les dispersions dans des phases liquides, les émulsions, les poudres, les solutions ; les pâtes et d'autres préparations à avaler ou à mâcher comme compléments alimentaires.

6. Préparation selon la revendication 4 ou 5,
**caractérisée en ce que** la concentration en trans-pellitorine, rapporté à la préparation finie, est comprise entre 0,1 ppm et 15 ppm.

7. Préparation selon l'une quelconque des revendications 4 à 6,
**caractérisée en ce que** celle-ci contient au moins une autre substance salivante, picotante, de saveur forte ou chaude.

8. Préparations selon l'une au moins des revendications 4 à 7,
**caractérisées en ce que** celles-ci contiennent au moins un extrait végétal salivant, picotant, de saveur forte ou chaude,

9. Préparations selon l'une au moins des revendications 4 à 8,
**caractérisées en ce qu'**il s'agit de trans-pellitorine synthétique,

10. Utilisation de la préparation servant à l'alimentation ou à la stimulation selon l'une quelconque des revendications 4 à 9, comme produit semi-fini pour l'aromatisation de préparations obtenues à partir d'elle comme produits finis, les produits finis ayant une concentration en trans-pellitorine dans l'intervalle de 0,01 à 20 ppm.
